# EUROPÄISCHE PATENTSCHRIFT

(11) **EP 2 421 623 B1**
(45) Veröffentlichungstag und Bekanntmachung des Hinweises auf die Patenterteilung: **02.11.2016**
(21) Anmeldenummer: 10714019.6
(22) Anmeldetag: 21.04.2010
(51) Int. Cl.: B01D 3/40

(54) **VERFAHREN ZUR TRENNUNG AROMATISCHER VERBINDUNGEN**
METHOD FOR SEPARATING AROMATIC COMPOUNDS
PROCÉDÉ DE SÉPARATION DE LIAISONS AROMATIQUES

(30) Priorität: 21.04.2009 EP 09158388
(43) Veröffentlichungstag der Anmeldung: 29.02.2012
(73) Patentinhaber: LANXESS Deutschland GmbH, 50569 Köln (DE)
(72) Erfinder: KUHLMANN, Sven, 50733 Köln (DE); WEBER, Hans-Martin, 51373 Leverkusen (DE); DUMMERSDORF, Hans-Ulrich, 51399 Burscheid (DE); HALLENBERGER, Kaspar, 51375 Leverkusen (DE); PFOHL, Oliver, 22587 Hamburg (DE)
(86) Internationale Anmeldenummer: PCT/EP2010/055297
(87) Internationale Veröffentlichungsnummer: WO 2010/122073

(56) Entgegenhaltungen:
- EP-A- 1 854 786
- WO-A-02/30878
- WO-A-02/074718
- WO-A-2005/019137
- DE-A1- 10 154 052
- DE-A1-102007 041 416
- US-A1- 2003 125 599
- KATO R ET AL: "Activity coefficients at infinite dilution of various solutes in the ionic liquids [MMIM]<+>[CH3SO4]<->, [MMIM]<+>[CH3OC2H4SO4]<->, [MMIM]<+>[(CH3)2PO4]<->, [C5H5NC2H5]<+>[(CF3SO2)2N]<-> and [C5H5NH]<+>[C2H5OC2H4OSO3]<->" FLUID PHASE EQUILIBRIA, ELSEVIER, Bd. 226, 10. Dezember 2004 (2004-12-10), Seiten 37-44, XP004917574 ISSN: 0378-3812
- ZHU JIQIN,YU YANMEI,CHEN JIAN, FEI WEIYANG: 'Measurement of activity coefficients at infinite dilution for hydrocarbons in imidazolium-based ionic liquids and QSPR model', [Online] Bd. FRONT. CHEM.ENG.CHINA 2007, Nr. I(2), 12 März 2007, HIGHER EDUCATION PRESS AND SPRINGER-VERLAG 2007, Seiten 190 - 194 DOI: 10.1007/s11705-007-0035-3 University of Beijing

## Beschreibung

Die Erfindung betrifft ein Verfahren zur destillativen Trennung von Stoffgemischen unter Einsatz von Ionischen Flüssigkeiten als Extraktionsmittel.

Die Rektifikation ist ein in der Technik verbreitet angewandte Methode um Stoffgemische voneinander zu trennen. Der erforderliche Aufwand für die Trennung eines Stoffgemisches bei der Rektifikation wird durch den sogenannten Trennfaktor a, dem Verhältnis der Verteilungskoeffizienten der beteiligten Komponenten wiedergegeben. Je höher der Abstand des Trennfaktors vom Wert eins ist, desto wirtschaftlicher lässt sich in der Regel das Stoffgemisch trennen.

Viele Stoffgemische, deren Komponenten nur geringe Siedepunktsunterschiede aufweisen oder gar Azeotrope bilden, sind durch konventionelle Rektifikation nur schwer bzw. gar nicht trennbar. Dies trifft auf die Trennung von Stellungsisomeren mehrfach substituierter aromatischer Verbindungen zu. In solchen Fällen wird dem zu trennenden Stoffgemisch typischerweise ein selektiver Zusatzstoff, ein sogenannter Entrainer zugesetzt, der über selektive Wechselwirkungen mit den Komponenten des Stoffgemisches das Dampf-Flüssig-Phasengleichgewicht in der Art beeinflusst, dass bessere Trennfaktoren erreicht werden, als ohne Zusatz der Entrainer. Typischerweise sinkt dadurch der Energie- bzw. Kostenaufwand für die Trennung eingesetzten Stoffgemisches. In jüngster Zeit sind auch ionische Flüssigkeiten als Entrainer Gegenstand von Untersuchungen geworden (siehe auch DE 10136614 A, DE 10154052 und Chem. Ing. Tech. 2003, 75, 1148-1149).

Aus der Literatur ist eine Vielzahl von Entrainern für die Trennung von aromatischen Verbindungen bekannt; z.B. Zhu Jiqin, Yu Yanmei, Chen Jian, Fei Weiyang; "Measurement of activity coefficients at infinite dilution for hydrocarbons in imidazolium-based ionic liquids and QSPR model" University of Beijing. Bd. Front. Chem. Eng. China 2007 Nr. 1(2) 12 März 2007 (2007-03-12), Seiten 190-194, Higher Education Press and Springer-Verlag 2007 DOI: 10.1007/s1175-007-0035-3.

Ferner beschreibt DE 10251191 A die Verwendung von Alkyl- und Alkylenphosphaten sowie Phosphinoxiden zur Trennung von Gemischen enthaltend m- und p-Dichlorbenzol, wobei Trennfaktoren von bis zu 1,23 erreicht werden. Weiterhin werden andere, allerdings weniger geeignete Entrainer offenbart.

EP 1 372 807 A beschreibt die Verwendung von ionischen Flüssigkeiten wie beispielsweise Imidazolium-tetrafluoroboraten, -hexafluorophosphaten, -trifluormethylsulfonylimiden und Methansulfonaten für die Trennung von aliphatischen Verbindungen.

Vor dem Hintergrund des vorstehend genannten Standes der Technik bestand die Aufgabe, ein Verfahren bereitzustellen, mit dem aroniatische Verbindungen besonders effizient getrennt werden können.

Gegenstand der Erfindung ist nun ein Verfahren zur Trennung von Mischungen aromatischer Verbindungen gemäß Anspruch 1.

Unter Ionischen Flüssigkeiten sind im Sinne der Erfindung Verbindungen zu verstehen, die mindestens ein Kation oder eine kationische Gruppe und mindestens ein Anion oder eine anionische Gruppe aufweisen, insgesamt jedoch ladungsneutral sind, und einen Schmelzpunkt unter 200°C aufweisen.

Bevorzugte Ionische Flüssigkeiten sind solche, die ein organisches Kation aufweisen.

Besonders bevorzugte Ionische Flüssigkeiten sind solche der Formel (I)

[K⁺] [A⁻] (I)

in der
[K⁺] für ein Kation steht, das ausgewählt ist aus der Gruppe der Formeln (IIa) bis (IId) 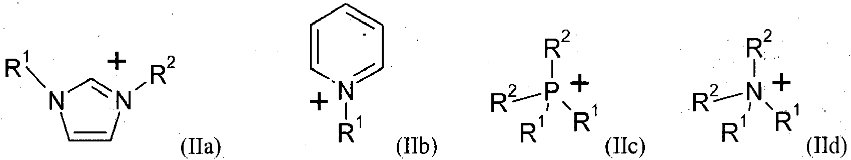 und
[A⁻] für ein Anion steht, das ausgewählt ist aus der Gruppe der Formeln (IIIa) bis (IIIg) 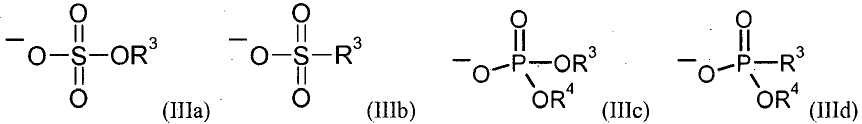 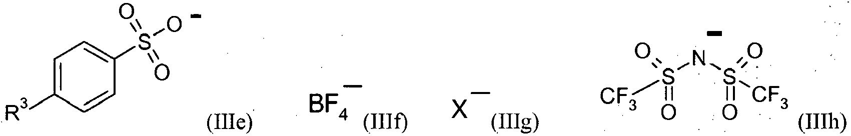 in denen
   jeder einzelne der Reste R¹ und R² sowie R³ und R⁴ jeweils völlig unabhängig voneinander für Wasserstoff, C₁-C₁₂-Alkyl-, C₅-C₁₄-Aryl, C₆-C₂₀-Arylalkyl -oder C₂-C₁₂-Alkylen stehen oder, soweit es die jeweilige Formel zulässt R¹ und R² beziehungsweise R³ und R⁴ zusammen und unabhängig von gegebenenfalls weiteren vorhandenen Resten R¹ und R² beziehungsweise R³ und R⁴ für C₂-C₁₂-Alkylen oder C₂-C₁₂-Alkenylen stehen.

Ganz besonders bevorzugte Ionische Flüssigkeiten sind solche der Formel (I) in denen [K⁺] für ein Kation der Formel (IIa) steht und [A⁻] für ein Anion der Formel (IIIc) steht wobei
R¹ und R² sowie R³ und R⁴ jeweils unabhängig voneinander für Wasserstoff, C₁-C₁₂-Alkyl-, C₅-C₁₄-Aryl, C₆-C₂₀-Arylalkyl -oder C₂-C₁₂-Alkylen stehen oder R¹ und R² bzw. R³ und R⁴ zusammen für C₂-C₁₂-Alkylen oder C₂-C₁₂-Alkenylen stehen.

C₁-C₁₂-Alkyl beziehungsweise C₂-C₁₂-Alkylen beziehungsweise C₂-C₁₂-Alkylen beziehungsweise C₂-C₁₂-Alkenylen bedeutet jeweils unabhängig einen geradkettigen, ganz oder teilweise cyclischen, verzweigten oder unverzweigten C₁-C₁₂-Alkyl beziehungsweise C₂-C₁₂-Alkyle beziehungsweise C₂-C₁₂-Alkylen beziehungsweise C₂-C₁₂-Alkenylen, wobei die genannten Reste gegebenenfalls weiterhin durch C₁-C₄-Alkoxyreste substituiert sein können.

Gleiches gilt für den nichtaromatischen Teil eines C₆-C₂₀-Arylalkyl-Restes.

C₁-C₁₂-Alkyl steht beispielsweise für Methyl, Ethyl, 2-Methoxyethyl, n-Propyl, iso-Propyl, n-Butyl, sec.-Butyl und tert.-Butyl, n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl, n-Octyl, Eldamantyl, die isomeren Menthyle, n-Nonyl, n-Decyl oder n-Dodecyl.

C₁-C₄-Alkoxy steht beispielsweise für Methoxy, Ethoxy, 2-Methoxyethoxy, n-Propoxy, iso-Propoxy, n-Butoxy, sec.-Butoxy oder tert.-Butoxy.

C₂-C₁₂-Alkenyl steht beispielsweise für Vinyl, 1-Propenyl, iso-Propenyl, 1-Butenyl, 2-Butenyl, 1-Pentenyl, 2-Pentenyl, 2-Methyl-l-butenyl, 2-Methyl-2-butenyl, 3-Methyl-l-buteny, 1-Hexenyl, 1-Heptenyl, 1-Octenyl oder 2-Octenyl.

C₅-C₁₄-Aryl bedeutet jeweils unabhängig einen heteroaromatischen Rest mit 5 bis 14 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, vorzugsweise jedoch für einen carbocyclischen aromatischen Rest mit 6 bis 14 Gerüstkohlenstoffatomen.

Bevorzuge Beispiele für carbocyclische aromatische Reste mit 6 bis 14 Gerüstkohlenstoffatomen sind zum Beispiel Phenyl, Naphtyl, Phenanthrenyl, Anthracenyl oder Fluorenyl, heteroaromatische Reste mit 5 bis 14 Gerüstkohlenstoffatomen in denen keines, ein, zwei oder drei Gerüstkohlenstoffatome pro Cyclus, im gesamten Molekül mindestens jedoch ein Gerüstkohlenstoffatoin, durch Heteroatome, ausgewählt aus der Gruppe Stickstoff, Schwefel oder Sauerstoff, substituiert sein können sind beispielsweise Pyridinyl, Oxazolyl, Benzofuranyl, Dibenzofuran-yl oder Chinolinyl.

Weiterhin kann der carbocyclische aromatische Rest oder heteroaromatische Rest mit bis zu fünf gleichen oder verschiedenen Substituenten pro Cyclus substituiert sein, die ausgewählt sind aus der Gruppe Chlor, Fluor, C₁-Cᵢ₂-Alkyl, C₁-C₁₂-Halogenalkyl und C₁-C₁₂-Alkoxy.

Besonders bevorzugt steht C₅-C₁₄-Aryl für Phenyl.

Gleiches gilt für den aromatischen Teil eines C₆-C₂₀-Arylalkyl-Restes. Bevorzugt steht C₆-C₂₀-Aryl für Benzyl.

Weiterer Gegenstand ist die Verwendung von Verbindungen der Formel (I) zur Trennung aromatischer Verbindungen.

Besonders bevorzugt steht jeder einzelne der Reste R¹, R², R³ und R⁴ jeweils unabhängig voneinander oder jeweils identisch für einen Rest aus der Reihe: Methyl, Ethyl, n-Propyl, n-Butyl, n-Hexyl, 2-methylhexyl oder n-Octyl.

Beispielhafte Verbindungen der Formel (I) sind 1-Methylimidazolium-hydrogensulfat, 1-Ethyl-3-Methylimidazolium-hydrogensulfat, 1,3-Dimethylimidazolium-methylsulfat, Ethyl-Pyridinium-tetrafluoroborat, 1,3-Dimethylimidazolium-methylhydrogenphosphat, 1,3-Dimethylimidazolium-dimethylphosphat, 1-Ethyl-3-methylinlidazolium-dimethylphosphat, 1,3-Diethyl-imidazolium-diethylphosphat, 1-Ethyl-3-methylimidazolium-diethylphosphat, 1-Methylimidazolium-methylhydrogenphosphonat, 1-Ethyl-3-methylimidazolium-metlrylsulfat, Tetrabutylphosphonium-chlorid, 1,3-Dimethylimidazolium-ethylsulfat, Allylimidazoliumbromid, 1-Ethyl-3-methylimidazolium-tosylat, Tributyl-tetradecylphosphonium-chlorid, 1-Butyl-4-methylpyridinium-tetrafluoroborat, 1-Butyl-3-methylimidazolium 2-(2-methoxyethoxy)ethyl-sulfat sowie 1-Hexyl-1-methylpyrrolidinium bis(trifluoromethylsulfonyl)imid.

Die Verbindungen der Formel (I) der Erfindung sind 1,3-Dimethylimidazoliuni-dimethylphösphat, 1-Ethyl-3-methylimidazolium-dimethylphosphat, 1,3-Diethyl-imidazolium-diethylphosphat und 1-Ethyl-3-methylimidazolium-diethylphosphat.

Die Verbindungen der Formel (I) und ihre Herstellung sind grundsätzlich bekannt und beispielsweise in Ionic Liquids in Synthesis, 2nd Ed., 2007, Wiley-VCH, Weinheim (Hrsg. Wasserscheid und Welton) offenbart.

Der Begriff aromatische Verbindungen umfasst im Sinne der Erfindung alle Verbindungen enthaltend cyclische aromatische Reste, die zu mindestens 95 % vorzugsweise zu mindestens 99 % thermisch unzersetzt bei irgendeiner Temperatur von 250°C oder weniger und irgendeinem Druck von mehr als 0,1 hPa, bevorzugt 0,5 hPa destilliert werden können.

C₅-C₁₄-Aryl bedeutet jeweils unabhängig einen heteroaromatischen Rest mit 5 bis 14 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, vorzugsweise jedoch für einen carbocyclischen aromatischen Rest mit 6 bis 14 Gerüstkohlenstoffatomen.

Bevorzuge Beispiele für carbocyclische aromatische Reste mit 6 bis 14 Gerüstkohlenstoffatomen sind zum Beispiel Phenyl, Naphtyl, Phenanthrenyl, Anthracenyl oder Fluorenyl, heteroaromatische Reste mit 5 bis 14 Gerüstkohlenstoffätomen.

Als Mischungen aromatischer Verbindungen eignen sich insbesondere Mischungen, die im Wesentlichen Stellungsisomere mehrfach substituierter aromatischer Verbindungen enthalten, wie beispielsweise Mischungen, die im Wesentlichen enthalten: m- und p-Dichlorbenzol; mund p-Kresol; m- und p-Xylol; m- und p- Chlortoluol; m- und p-Nitrotoluol; m- und p-Nitrochlorbenzol; m- und p-Trifluormethoxynitrobenzol, 1,2,3- und 1,2,4-Trichlorbenzol; 2,4-und 2,5-Dichlortoluol; 2,4- und 2,6-Dichlortoluol; 2,5- und 2,6-Dichlortoluol; 2,4- und 2,6-Dinitrotoluol; 2,3- und 3,4-Dichlomitrobenzol; 2,3- und 3,4-Dichloranilin oder Styrol und Ethylbenzol.

Die Mischungen der Erfindung sind Mischungen, die im Wesentlichen m- und p-Dichlorbenzol enthalten.

Das jeweilige Verhältnis der zu trennenden Komponenten im Ausgangsgemisch ist dabei prinzipiell nicht begrenzt. Der Begriff "im Wesentlichen" soll hierbei bedeuten, dass der Anteil sonstiger Komponenten über die zwei zu trennenden Komponenten hinaus 0 bis 20, bevorzugt 0 bis 10 Gew.-% und besonders bevorzugt 0 bis 5 Gew.-% betragen kann.

Das erfindungsgemäße Verfahren betrifft Trennung von m- und p-Dichlorbenzol.

Im Allgemeinen wird das erfindungsgemäße Verfahren so durchgeführt, dass die in die Extraktionskolonne eingespeiste, zu trennende Mischung auf eine Temperatur gebracht wird oder aufweist die in Abhängigkeit vom gewählten Kolonnendruck zwischen 20 und 150°C und vorzugsweise zwischen 80 und 120°C liegt. In einer bevorzugten Ausfuhrungsform liegt die Sumpftemperatur in der Rektifikationskolonne bei 180°C oder weniger, besonders bevorzugt bei 130°C oder weniger.

In einer bevorzugten Ausuhrungsform wird die Trennung in einer Rektifikationskolonne durchgeführt, wobei der Druck am Kolonnenkopf im Allgemeinen und abhängig vom zu trennenden Gemisch beispielsweise im Bereich von 5 bis 500 hPa und bevorzugt 5 bis 200 hPa betragen kann.

Die Druckdifferenz zwischen Kolonnensumpf und Kolonnenkopf kann beispielsweise 0 bis 200, bevorzugt 0 bis 100 hPa betragen.

Die Anzahl der theoretischer Trennstufen kann beispielsweise 5 bis 1000, vorzugsweise 20 bis 200 betragen.

Das Gewichtsverhältnis von Rücklauf zu Destillatentnalune kann beispielsweise 0,5:1 bis 20:1, besonders bevorzugt 2:1 bis 8:1 betragen.

Das Gewichtsverhältnis von Verbindungen der Formel (I) zur Menge des zu trennenden Stoffgemisches, das der Rektifikationskolonne zugeführt wird, kann beispielsweise 2:1 bis 40:1, bevorzugt 2:1 bis 8:1 betragen.

Zur Rückgewinnung der Verbindungen der Fonnel (I) erfolgt zum Beispiel durch destillative Abtrennung vom restlichen Produkt. Vorzugsweise erfolgt die destillative Abtrennung in zwei Trennstufen ohne Rücklauf. Dabei wird in der ersten Trennstufe bei einem Druck im Bereich von 5 bis 200 hPa und bevorzugt 5 bis 50 hPa das restliche Produkt weitestgehend abgetrennt, wobei unter dem Begriff "weitestgehend" hierbei eine mehr als 90%-ige Abtrennung, vorzugsweise eine mehr als 95%-ige Abtrennung zu verstehen ist. In einer zweiten Trennstufe erfolgt danach eine weitere Abtrennung bei einem niedrigeren Druck als in der ersten Trennstufe von beispielsweise 1 bis 20 hPa und bevorzugt 1 bis 5 hPa. Die Kondensation der Produktbrüden findet zweckmäßigerweise nach einer Entspannung auf beispielsweise 5 bis 200 hPa und bevorzugt 5 bis 50 hPa statt.

Der besondere Vorteil des erfindungsgemäßen Verfahrens ist, dass in effizienter Weise Mischungen aromatischer Verbindungen derart voneinander getrennt werden können, dass die einzelnen Komponenten mit hohen Reinheiten erhalten werden können.

### Beispiele

### Trennung von Dichlorbenzol-Gemischen

Folgende Koeffizienten von Aktivitätsfaktoren wurden mit Hilfe des Programms COSMO-RS ermittelt, das dem Fachmann anerkanntermaßen zur Vorhersage von thermodynamischen Stoffdaten verhilft:

| **Kation** | **Anion** | **γ (m-DCB)/ γ(p-DCB) @150°C Cosmo-RS*** |
|---|---|---|
| 1-Methylimidazolium | HSO₄ | 1,24 |
| 2-Ethylimidazolium | HSO₄ | 1,20 |
| 1,3-Dimethylimidazolium | MeSO₄ | 1,20 |
| N-Ethylpyridinium | BF₄ | 1,19 |
| 1,3-Dimethylimidazolium | MeHPO₄ | 1,19 |
| 1,3-Dimethylimidazolium | Me₂PO₄ | 1,18 |
| 1,3-Dimethylimidazolium | MeHPO₃ | 1,18 |
| 1-Ethyl-3-methylimidazolium | MeSO₃ | 1,18 |
| Tetra-n-butylphospbonium | Cl | 1,17 |
| 1-Ethyl-3-methylimidazolium | EtSO₄ | 1,17 |
| 1-Allylimidazolium | Bromid | 1,17 |
| 1-Ethyl-3-methylimidazolium | Tosylat | 1,16 |
| Tributyl-tetradecylphosphonium | Chlorid | 1,16 |
| 1,3-Diethyl-imidazolium | Et₂PO₄ | 1,16 |
| 1-Butyl-4-methylpyridinium | BF₄ | 1,14 |
| 1-Butyl-3-methylimidazolium | MDEGSO4₄ | 1,14. |

| | | |
|---|---|---|
| *unendliche Verdünnung | | |

Daten zur Wirksamkeit der erfindungsgemäßen Extraktionsmittel sind in nachfolgender, Tabelle zusammengestellt. Diese Trennfaktoren wurden bei 120°C nach der Headspacemethode gemessen (siehe auch Verfahrenstechnik 8 (1974), 12, 343-347)

Die Messungen wurden bei einer Konzentration in der Flüssigkeit von 10 Mol-% m-Dichlorbenzol, 10 Mol% p-Dichlorbenzol und 80 Mol-% Extraktionsmittel ausgeführt. Zum Vergleich der Wirksamkeit dieser Extraktionsmittel sind auch die entsprechenden Messwerte von Triethylphosphat und Sulfolan in der Tabelle aufgenommen worden.

| Extraktionsmittel | Trennfaktor |
|---|---|
| 1,3-Diethylinvdazolium-diethylphosphat | 1,34 |
| 1,3-Ethylmethylimidazolium-dimethylphosphat | 1,31 |
| 1,3-Dimethylimidazolium-dimethylphosphat | 1,29 |
| 1,3-Ethlymethylimidazolium-diethylphosphat | 1,27 |
| Triethylphosphat | 1,23 |
| Sulfolan | 1,14 |

Die Prüfungen zeigen, dass die erfindungsgemäßen Extraktionsmittel im Vergleich zum Stand der Technik ebenbürtig oder überlegen sind.

Weitere Untersuchungen haben gezeigt, dass die erfindungsgemäßen Extraktionsmittel eine hohe thermische Stabilität zeigen auch in Gegenwart von Rost und anderen Schwermetallverbindungen.

### Destillaticinsbeispiel

Eine Extraktionskolonne mit 100 theoretischen Trennstufen wurde mit dem zu trennenden Gemisch bestehend aus 58 Gew.-% m-Dichlorbenzol und 38 Gew.-% p-Dichlorbenzol mit einer Temperatur von 100°C und dem Extraktionsmittel 1,3-Diethylimidazoliumdiethylphosphat mit einer Temperatur von 60°C gespeist. Dabei wurde der Entrainer oberhalb der des Dichlorbenzol-Gemisches eingespeist. Das Verhältnis von Dichlorbenzol zu Entrainer lag bei 1:5.

Der Druck am Kolonnenkopf betrugt 100 hPa und im Kolonnensumpf 140 hPa. Das Extraktionsmittel verliess den Kolonnensumpf mit einer Temperatur von 158°C. Im Sumpfstrom waren mehr als 99 Gew.-% des eingesetzten p-Dichlorbenzol und weniger als 50 Gew.-% des eingesetzten m-Dichlorbenzol enthalten. Im Kopfstrom der Kolonne wurde hochreines Zielprodukt mit einem Gehalt von mehr als 99 Gew.-% m-Dichlorbenzol entnommen, wobei das Rücklaufverhältnis (Rückflussmenge/Destillat) bei 6:1 lag.

## Patentansprüche

1. Verfahren zur Trennung von Mischungen aromatischer Verbindungen, die im wesentlichen Stellungsisomeren mehrfach substituierter aromatischer Verbindungen ausgewählt aus der Gruppe m- und p-Dichlorbenzol enthalten mittels extraktiver Rektifikation, **dadurch gekennzeichnet, dass** die Rektifikation in Gegenwart einer oder mehrerer Ionischer Flüssigkeiten ausgewählt aus der Gruppe: 1,3-Dimethylimidazolium-dimethylphosphat, 1-Ethyl-3-methylimidäzolium-dimethylphosphat, 1,3 Diethyl-imidazolium-diethylphosphat und 1Ethyl-3-methylimidazolium-diethylphosphat durchgeführt wird.

2. Verfahren nach Anspruch 1, **dadurch gekennzeichnet, dass** die sumpftemperatur in der extraktiven Rektifikation 180°C oder weniger beträgt.

3. Verfahren nach einem der Ansprüche 1 bis 2, **dadurch gekennzeichnet, dass** die Anzahl der theoretischer Trennstufen bei der extraktiven Rektifikation 5 bis 1000 beträgt.

4. Verfahren nach einem der Ansprüche 1 bis 3, dadurch gekenntzeichnet, dass das Gewichtsverhältnis von Rücklauf zu Destillatentnahme bei der extraktiven Rektifikation 0,5:1 bis 20:1 beträgt.

## Claims

1. Method of separating mixtures of aromatic compounds containing essentially positional isomers of multiply substituted aromatic compounds selected from the group consisting of m- and p-dichlorobenzene by means of extractive rectification, **characterized in that** the rectification is carried out in the presence of one or more ionic liquids selected from the group consisting of: 1,3-dimethylimidazolium dimethylphosphate, 1-ethyl-3-methylimidazolium dimethylphosphate, 1,3-diethyl-imidazolium diethylphosphate and 1-ethyl-3-methylimidazolium diethylphosphate.

2. Method according to Claim 1, **characterized in that** the temperature at the bottom in the extractive rectification is 180°C or less.

3. Method according to either of Claims 1 to 2, **characterized in that** the number of theoretical plates in the extractive rectification is from 5 to 1000.

4. Method according to any of Claims 1 to 3, **characterized in that** the weight ratio of runback to distillate taken off in the extractive rectification is from 0.5:1 to 20:1.

## Revendications

1. Procédé de séparation de mélanges de composés aromatiques, qui contiennent principalement des isomères de position de composés aromatiques polysubstitués choisis dans le groupe du m- et p-dichlorobenzène, par rectification extractive, **caractérisé en ce que** la rectification est réalisée en présence d'un ou de plusieurs liquides ioniques choisis dans le groupe constitué par le diméthylphosphate de 1,3-diméthylimidazolium, le diméthylphosphate de 1-éthyl-3-méthylimidazolium, le diéthylphosphate de 1,3-diéthylimidazolium et le diéthylphosphate de 1-éthyl-3-méthylimidazolium.

2. Procédé selon la revendication 1, **caractérisé en ce que** la température de fond dans la rectification extractive est de 180 °C ou moins.

3. Procédé selon l'une quelconque des revendications 1 à 2, **caractérisé en ce que** le nombre d'étapes de séparation théoriques lors de la rectification extractive est de 5 à 1 000.

4. Procédé selon l'une quelconque des revendications 1 à 3, **caractérisé en ce que** le rapport en poids entre le reflux et le soutirage de distillat lors de la rectification extractive est de 0,5:1 à 20:1.
